(19) Europäisches Patentamt
European Patent Office
Office européen des brevets

(11) Veröffentlichungsnummer: **0 060 407**
**B1**

# (12) EUROPÄISCHE PATENTSCHRIFT

(45) Veröffentlichungstag der Patentschrift: **12.12.84**

(51) Int. Cl.³: **C 12 N 1/00**, C 12 N 1/20, A 23 K 1/00, C 05 F 11/08

(21) Anmeldenummer: **82101265.5**

(22) Anmeldetag: **19.02.82**

## (54) Verfahren zur Herstellung von penicillinfreien Myzelmassen aus fermentativ gebildeten Penicillinproduktionskulturen und ihre Verwendung als Tierfutter und Düngemittel.

(30) Priorität: **23.02.81 DE 3106649**

(43) Veröffentlichungstag der Anmeldung: **22.09.82 Patentblatt 82/38**

(45) Bekanntmachung des Hinweises auf die Patenterteilung: **12.12.84 Patentblatt 84/50**

(84) Benannte Vertragsstaaten: **AT DE FR GB IT NL SE**

(56) Entgegenhaltungen:
**BE - A - 505 453**
**DD - A - 139 083**
**DE - B - 1 103 735**
**DE - B - 1 245 271**
**GB - A - 649 818**
**US - A - 3 928 642**

(73) Patentinhaber: **HOECHST AKTIENGESELLSCHAFT, Postfach 80 03 20, D-6230 Frankfurt am Main 80 (DE)**

(72) Erfinder: **Ebert, Hildegard, Sodener Weg 24A, D-6232 Bad Soden am Taunus (DE)**
Erfinder: **Kreutzfeldt, Richard, Dr., Geisenheimer Strasse 130, D-6000 Frankfurt am Main 71 (DE)**

**EP 0 060 407 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist ein Verfahren zur Herstellung von penicillinfreien Myzelmassen unter Entfernung der Penicillinreste aus fermentativ gebildeten Penicillinproduktionskulturen durch anaeroben bakteriellen Aufschluß unter konservierenden Silierungsbedingungen. Ferner die Verwendung des resultierenden Silageprodukts als Tierfutter, insbesondere als Einzelfuttermittel für Schweine und Rinder, sowie als Düngemittel.

Bei der mikrobiologischen Gewinnung des Antibiotikums Penicillin entstehen während der Fermentation große Mengen an Penicillium-Myzel als lästiges Nebenprodukt. Penicillium-Myzel ist die Pilzsubstanz des Penicillinbildners, Penicillium chrysogenum. Es wird im Penicillingewinnungsprozeß z. B. durch Filtration oder Zentrifugation von der Kulturlösung abgetrennt und enthält, je nach Wirksamkeit des angewandten Waschprozesses, noch unterschiedliche Mengen an Restpenicillin. Im Filtrat der Kulturlösung befindet sich das Penicillin, das durch Extraktion der Lösung gewonnen wird.

Einer Beseitigung oder Nutzung der bei diesen Fermentationsverfahren zwangsläufig anfallenden Myzelmassen steht vor allem deren Restpenicillingehalt entgegen, dessen schadlose Entfernung eine wichtige Voraussetzung für die meisten Deponier- bzw. Verwertungsmöglichkeiten ist. Die mit einem Wassergehalt von etwa 80—90 Gew.-% in meist stichfester Form anfallende Menge an sogenannter Naßmyzel-Masse beträgt ca. 25—35 t pro 100 cbm Kulturlösung. Sie kann durch intensives Nachpressen, z. B. auf einer Bandpresse, auf etwa 25 bis 30 Gew.-% Trockenmasse gebracht werden. Die Trockenmasse (=TM) hat im Mittel z. B. folgende Zusammensetzung:

91 Gew.-% organische Substanz, davon 44 Gew.-% Rohprotein,
9 Gew.-% anorganische Substanz,
der Restpenicillingehalt beträgt ca. 2000—5000 mg/kg TM.

Wegen seines hohen Wassergehaltes ist das anfallende Rohmyzelprodukt, das vorzugsweise etwa 10 bis 30 Gew.-% Trockenmasse enthält, sehr leicht verderblich. Bereits nach einem Tag beginnt es infolge rasch einsetzender Fäulnis in eine übelriechende Masse überzugehen. Dies bedeutet, daß das Myzel kurzfristig beseitigt oder einer geeigneten Verwendung zugeführt werden muß.

In früheren Jahren, als die mikrobiologische Penicillin-Produktion in verhältnismäßig kleinen Dimensionen erfolgte, fanden sich noch gangbare Wege, um das Myzel ohne große Schwierigkeiten zu beseitigen, z. B. durch Deponie auf Abfallhalden oder durch Abgabe als Viehfutter. In jüngster Zeit ist eine solche Beseitigung jedoch nicht mehr möglich. Eine Deponie auf Abfallhalden verbietet sich wegen der in modernen Großanlagen anfallenden sehr großen Myzelmengen, z. B. ca. 30 t täglich, und der daraus erwachsenden starken Geruchsbelästigung. Das gesamte Myzelprodukt muß daher aus Gründen der Umweltbelastung auf andere Weise beseitigt werden, z. B. indem man es in starker Verdünnung durch eine biologische Kläranlage leitet, was jedoch mit einem enormen Kostenaufwand verbunden ist.

Auch eine Verwertung als Viehfutter ist heute wegen des Restgehaltes an Penicillin unzulässig.

Es sind bereits Versuche gemacht worden, das Myzel wegen seines Stickstoffgehaltes zur Düngung in der Landwirtschaft einzusetzen. Schwierigkeiten bereitet es jedoch, ausreichend große Flächen zu finden, die Tag für Tag über das ganze Jahr verfügbar sind. Eine Geruchsbelästigung ist auch hier nicht auszuschließen und es besteht bei noch vorhandenen Restpenicillingehalten die Gefahr einer möglichen Ausbildung von Penicillinresistenzen bei Mensch und Tier.

In der DD-PS 139 083 ist ein Verfahren zur Herstellung eines penicillinfreien Myzeltrockenprodukts, das z. B. als Düngemittel oder Tierfutter verwertbar ist, beschrieben, bei dem die auf einen pH-Wert von <5 angesäuerte wäßrige Myzelsuspension der Zerstäubungstrocknung bei 150 bis 300° C unterworfen und der Penicillingehalt dadurch zerstört wird.

In der US-PS 3 928 642 ist ein Verfahren zur Zerstörung des Restpenicillins durch Erhitzen der Myzelmassen auf 140—200° C unter Druck und anschließender Trocknung sowie Verwendung des Verfahrensproduktes als Tierfutter beschrieben.

Beide Verfahren sind jedoch apparativ aufwendig, erfordern hohe Energiekosten und sind für die Praxis in hohem Maße unwirtschaftlich.

Der vorliegenden Erfindung lag somit die Aufgabe zugrunde, die großtechnisch anfallenden Myzelmassen auf einfache und wirtschaftliche Weise von ihrem Restpenicillingehalt zu befreien und das Myzelprodukt mit seinem wertvollen Proteingehalt in einer Qualitätsform zu erhalten, die dessen möglichst vollständige weitere Verwertung, z. B. als preisgünstiges Tierfutter, insbesondere in der Nutzviehmast, oder als Düngemittel ermöglicht.

Es wurde nun überraschenderweise gefunden, daß man den Restpenicillingehalt in fermentativ gebildeten, wasserhaltigen Penicillinproduktionskulturen (=Naßmyzel) praktisch vollständig eliminieren kann, wenn man die Penicillin-Rohmyzelmassen, die übliche Mengen an Restpenicillin, vorzugsweise etwa 2000 bis 5000 mg Penicillin pro kg Trockenmasse enthalten, einer anaeroben Milchsäuregärung unter Verwendung von penicillinresistenten Lactobazillen unterwirft, in deren Verlauf die Myzelmasse aufgeschlossen und in ein Silageprodukt umgewandelt wird.

Es wurde außerdem gefunden, daß man Lactobazillen, die an sich penicillin-empfindlich sind, z. B.

dadurch an Penicillin gewöhnen kann, daß man sie in Teilmengen der Rohmyzelmasse, die übliche Mengen an Restpenicillin enthält, unter Zusatz von fermentativ in Milchsäure umwandelbaren Zucker anaerob züchtet. Dabei bauen sie in einer Milchsäuregärung unter Aufschluß der Myzelmasse und teilweiser Zerstörung der schlauchartig aussehenden Myzelstrukturen das Restpenicillin ab und man erhält ein penicillinfreies, milchsaures und dadurch haltbar gewordenes Myzelsilageprodukt, das penicillinresistente Lactobazillen enthält und sich hervorragend als Impfstoff für die penicillinabbauende, anaerobe Milchsäuregärung von weiteren Rohmyzelsilierungsmassen eignet, in deren Verlauf sich die Bildung von penicillinresistenten Lactobazillen fortsetzt.

Für die Gewinnung von penicillinresistenten Lactobazillen kann man z. B. von solchen bekannten bakteriellen Milchsäurebildnern ausgehen, wie sie bevorzugt in den anaerob unter Milchsäuregärung hergestellten landwirtschaftlichen Gärfuttersilagen, beispielsweise Grassilagen, Grünmaissilagen, Rübenblättersilagen etc. in großen Mengen vorhanden sind, indem man diese Silageprodukte bzw den Silagesaft als Lactobazillus-Impfstoff einsetzt, oder aber wie sie auch in der uns umgebenden atmosphärischen Luft in meist ausreichender Menge allgegenwärtig sind, indem man die zu beimpfenden Rohmyzelteilmengen einige Stunden offen an der Luft stehen läßt, z. B. als Filterkuchenmasse offen neben der Filterpresse im Filtrationsbetrieb lagert.

Gegenstand der vorliegenden Erfindung ist daher ein Verfahren zur Herstellung von penicillinfreien Myzelmassen aus fermentativ gebildeten, wasserhaltigen Penicillinproduktionskulturen (=Naßmyzel), die übliche Mengen an Restpenicillin enthalten, unter Eliminierung des Restpenicillins, dadurch gekennzeichnet, daß man die Restpenicillin enthaltende Penicillium-Rohmyzelmasse einer anaeroben Milchsäuregärung unter Verwendung penicillinresistenter Lactobazillen unterwirft.

Als Variante des vorstehenden Hauptverfahrens wird weiterhin ein Verfahren beansprucht, das dadurch gekennzeichnet ist, daß man zunächst in einer Teilmenge der Penicillium-Rohmyzelmasse unter an sich bekannten Bedingungen eine anaerobe Milchsäuregärung durchführt, wobei penicillinresistente Lactobazillen erhalten werden und die Rohmyzelmasse in ein penicillinfreies Myzelsilageprodukt umgewandelt wird, welches man anschließend als Impfstoff der Hauptmenge der Rohmyzelmasse zuführt und diese einer anaeroben Milchsäuregärung entsprechend dem Hauptverfahren unterwirft.

Die Milchsäuregärung führt erfindungsgemäß zu einer deutlichen Verflüssigung des ursprünglich stichfesten Rohmyzel-Ausgangsproduktes unter Bildung einer pumpfähigen Silagemasse und infolge der Milchsäurebildung zur Erniedrigung ihres pH-Wertes auf Werte $<4,5$, vorzugsweise 4,2 bis 4,5, insbesondere 4,4, woraus eine konservierende Wirkung auf das meist ockerfarbig aussehende Myzelsilageprodukt resultiert, die letzteres bei Normaltemperatur unter anaeroben Bedingungen, z. B. in einem Silageturm, mehrere Monate lagerfähig erhält. Das erfindungsgemäß hergestellte Silageprodukt besitzt einen angenehmen, leicht säuerlichen, hefig-aromatischen Geruch. Es wird von Tieren, insbesondere von Nutztieren, vorzugsweise von Mastrindern und Mastschweinen gern gefressen und führt insbesondere als eiweißreicher Futtermittelbestandteil bzw. als Einzelfuttermittel in Verbindung mit anderen, energiereichen Futtermitteln zu ausgezeichneten Mastergebnissen. Das Myzelsilageprodukt enthält u. a. die Aminosäuren Lysin, Methionin und Cystein.

Gegenstand der Erfindung sind daher auch Futtermittel mit einem Gehalt an erfindungsgemäß hergestelltem penicillinfreien Myzelsilageprodukt sowie deren Verwendung als Tierfutter, insbesondere Nutztierfutter, vorzugsweise als Rinder- und Schweinemastfutter.

Ein weiterer Gegenstand der Erfindung ist auch ein Verfahren zum Mästen von Rindern und Schweinen, das dadurch gekennzeichnet ist, daß man die Masttiere in üblicher Weise mit Mischfuttermitteln, die erfindungsgemäß hergestelltes penicillinfreies Myzelsilageprodukt enthalten, füttert.

Da die erfindungsgemäß hergestellten penicillinfreien Myzelsilageprodukte bisher weder bekannt noch vorbeschrieben sind, werden sie als solche von dem Gegenstand der vorliegenden Erfindung ebenfalls mit umfaßt.

Eine weitere vorteilhafte Verwendbarkeit erfindungsgemäßer penicillinfreier Myzelsilageprodukte besteht darin, daß man sie, insbesondere wegen ihres Stickstoffgehaltes, als universell verwendbare und umweltfreundliche Düngemittel einsetzt, z. B. im Obstbau, Gartenbau und Weinbau, in der Land- und Forstwirtschaft sowie zur Steigerung erwünschter Umweltvegetation. Dabei können die flüssige Konsistenz und die Pumpfähigkeit der Produkte beim Ausbringen und Verteilen von erheblichem Vorteil sein, u. a. indem man sie z. B. über Rohrleitungen, auch an Hanglagen oder in Gewächshäusern, transportieren und über Regner auf dem Boden verteilen kann.

Die erfindungsgemäße Verfahrensweise läßt sich auf alle bekannten und fermentativ gebildeten Penicillinproduktionsmyzelien anwenden. Bevorzugt sind diejenigen aus der Penicillin G- und V-Produktion. Besonders bevorzugt sind die Penicillium-Myzelien der Penicillin G-Produktion.

Wie entsprechende Analysen gezeigt haben, läßt sich in den erfindungsgemäß hergestellten Myzelsilageprodukten auch mit den empfindlichsten Untersuchungs- und Nachweismethoden kein Penicillin mehr nachweisen.

Dieses Ergebnis ist um so überraschender, als es dem Fachmann seit langem bekannt ist, daß von Lactobazillen bewerkstelligte Milchsäuregärungen, insbesondere z. B. verschiedene im Molkereigewerbe durchgeführte Gärungen, durch Penicillin praktisch vollständig gehemmt werden können (vgl. z. B. Max Schultz »Das große Molkereilexikon«, Volkswirtschaftlicher Verlag Kempten, Bd. II, 4. Aufl. (1965), S. 895). Das erfindungsgemäße Verfahren ist darüber hinaus in seiner Wirtschaftlichkeit allen bisher bekanntgewordenen Myzelienaufbereitungsverfahren deutlich überlegen.

Das erfindungsgemäße Silierungsverfahren kann sowohl chargenweise, z. B. in Gärfässern oder in Silagetürmen, als auch kontinuierlich, z. B. in Silagetürmen, die zylinderförmig sein können, welche man von oben beschickt und denen man das fertige Silageprodukt über eine Ablaßvorrichtung, die sich in dem z. B. konisch verjüngten Bodenteil befinden kann, kontinuierlich bzw. in gewissen Zeitabständen entnimmt. Die kontinuierliche Verfahrensweise, die bevorzugt ist, macht eine Mindestverweilzeit in dem Silierungsbehälter erforderlich, die durch den Zeitbedarf für den Ablauf der gewünschten Milchsäuregärung unter den jeweils eingehaltenen Verfahrensbedingungen, wie z. B. Temperatur, Zuckergehalt, Restpenicillingehalt, Bakterienaktivität, bestimmt wird. Sie kann z. B. in einem 12 cbm fassenden Silierturm unter günstigen Bedingungen bei 20—25° C ca. 6 Tage betragen. Soll das gewonnene Myzelsilageprodukt anschließend gelagert werden, so muß dessen Lagerung unter anaeroben Bedingungen erfolgen, wobei die Lagerungstemperatur vorteilhaft die Normaltemperatur nicht übersteigen soll.

Als fermentativ in Milchsäure umwandelbare Zucker kommen bevorzugt solche Produkte zur Anwendung, wie sie z. B. auch bei der landwirtschaftlichen Futtermittelsilierung eingesetzt werden. Es sind dies insbesondere Melassen aus der Saccharose- oder Glucoseherstellung (Zuckergehalt ca. 50 Gew.-%), Hexosen aus Kartoffel- oder Maisstärke, Glucose, Lactose, Saccharosen wie Rohrzucker oder Rübenzucker, Invertzucker, Traubenzucker. Aber auch alle übrigen bekannten Zucker und zuckerhaltigen Abfallprodukte, beispielsweise melassierte Zuckerrübentrockenschnitzel, Zuckergemische und Zuckersirupprodukte bzw. gegebenenfalls auch deren Derivate, sofern diese sich fermentativ in Milchsäure umwandeln lassen, oder deren wäßrige Lösungen oder Verdünnungen können eingesetzt werden. Besonders bevorzugt sind Melassen.

Der Zuckeranteil in der für die anaerobe Milchsäuregärung eingesetzten Rohmyzelmasse beträgt vorzugsweise etwa 5 bis 10 Gew.-%, bezogen auf die Rohmyzel-Trockenmasse, wobei der Anteil nach oben hin nicht kritisch ist und bei seiner Wahl im allgemeinen Wirtschaftlichkeitsgesichtspunkte im Vordergrund stehen. Dagegen kann die untere Grenze des Zuckeranteils nicht beliebig weit gesenkt werden. Sie muß vielmehr in einer Mindestgrößenordnung liegen, die eine Milchsäuregärung in solchem Umfang ermöglicht, daß durch die gebildete Milchsäure die resultierende Myzelsilage einen pH-Wert $<4,5$ annimmt und dadurch haltbar und lagerungsfähig wird. Bei zu niedrigen Zuckergehalten besteht die Gefahr, daß sich zu wenig Milchsäure bildet, daß Fehlgärungen, z. B. unter Bildung von Buttersäure und Essigsäure stattfinden können und daß der Restpenicillingehalt nicht vollständig abgebaut wird, so daß die Silage verdirbt und zum Verfüttern unbrauchbar ist. Es hat sich daher als vorteilhaft erwiesen, daß die eingesetzte Rohmyzelmasse vorzugsweise mindestens 1,5 Gew.-%, insbesondere 1,5 bis 5 Gew.-% Zucker, bezogen auf das Gesamtgewicht der wasserhaltigen Rohmyzelmasse, enthält (entsprechend z. B. etwa 3 bis 10 Gew.-% Melasse). Es hat sich weiterhin gezeigt, daß man beim erstmaligen Züchten von penicillinresistenten Lactobazillus-Kulturen, die später als Impfstoff eingesetzt werden, den Bakterien vorteilhaft möglichst hohe Zuckernährstoffgehalte anbietet und dazu mit Zuckergehalten im oberen Teil des angegebenen Bereichs arbeitet. Vorzugsweise setzt man hierbei 3,5 bis 5 Gew.-% Zucker, bezogen auf das Gesamtgewicht der wasserhaltigen Rohmyzelmasse, bzw. 25 bis 35 Gew.-% Zucker, bezogen auf die Rohmyzel-Trockenmasse, ein.

Der erfindungsgemäße Prozeß der anaeroben Milchsäuregärung unter Myzel-Aufschluß, Abbau des Restpenicillins und konservierender Säuerung der Myzelsilage verläuft bei Temperaturen von wenig unterhalb bis zu leicht oberhalb der Normaltemperatur liegenden Werten, vorzugsweise bei etwa 20 bis 25° C. Nach Einsetzen der Gärung kann unter günstigen Gärungsbedingungen bereits nach 2 bis 3 Tagen ein im Bereich zwischen 4,5 und 4,2 liegender pH-Wert erreicht werden, wodurch das Sauerprodukt unter anaeroben Bedingungen und Normaltemperatur, z. B. in einem Silageturm, einige Monate lagerungsfähig bleibt. Als Gesamtgärzeit sind selbst unter günstigen Gärbedingungen jedoch mindestens 6 Tage erforderlich, um einen vollständigen Abbau des Restpenicillins erzielen zu können.

Wie unter dem Mikroskop zu erkennen ist, verläuft die erfindungsgemäße Myzelsilierung unter teilweiser Zerstörung der schlauchförmig aussehenden Myzelstrukturen. Durch diesen Myzelaufschluß wird das Silageprodukt, vergleichsweise zum rohen Ausgangsmyzel, in der Tierfütterung besser verdaulich, von den Tieren besser angenommen und als Futtermittel somit besser verwertbar.

Es hat sich außerdem gezeigt, daß es möglich ist, der eingesetzten Rohmyzelmasse anteilig auch solche silierbaren Futtermittelbestandteile zuzusetzen, wie sie bei der üblichen Herstellung von Gärfuttersilage eingesetzt werden, beispielsweise Gras, Grünmais, Rübenblätter, Rübenschnitzel und insbesondere Getreidestroh, vorzugsweise in gut gehäckselter Form, um die Pumpfähigkeit des resultierenden Myzelsilageproduktes ggfs. zu erhalten. Die Zusatzmenge solcher Futtermittelbestandteile ist an sich nicht kritisch. Sie beträgt gegebenenfalls vorzugsweise bis zu maximal 15 Gew.-%, bezogen auf das Gesamtgewicht der wasserhaltigen Rohmyzelmasse, wenn das zu bildende Silageprodukt in pumpfähiger Form erhalten werden soll.

Fütterungsversuche an Schweinen und Rindern haben ergeben, daß diese das erfindungsgemäß silierte Myzelprodukt gerne fressen, wobei Rinder ggfs. eine kurze anfängliche Gewöhnungsphase benötigen. Die Versuchsrinder zeigten außerdem etwas bessere Gewichtszunahmen als die Kontrollgruppe. Die Verdaulichkeit des Produktes ist sehr gut.

Erfindungsgemäße Myzelsilageprodukte werden besonders bevorzugt als preisgünstige, eiweißreiche Einzelfuttermittel in Verbindung mit üblichen anderen, energiereichen Futtermitteln und ggfs. Ergänzungsfuttermitteln in der Schweine- und Rindermast verwendet, wobei ausgezeichnete Mastergebnisse erzielt werden können.

Die Erfindung wird durch die nachfolgenden Beispiele näher erläutert, ohne sich jedoch auf diese zu beschränken.

## Vergleichsbeispiel 1

1 kg Penicillium-Naßmyzel (stichfest) aus der Penicillin G-Produktion (TM = 11 Gew.-%, Penicillin G-Gehalt = 2350 mg/kg TM) wird ohne Fremdinfektion mit 30 g steriler Stärkezuckerlösung (Glucosegehalt = 50 Gew.-%) unter sterilen Bedingungen gemischt und das Gemisch in einem sterilen 2 l-Erlenmeyerkolben bei Raumtemperatur (23° C) unter anaeroben Bedingungen stehengelassen, wobei der Rest-Sauerstoff in dem Kolben durch Stickstoffüberlagerung entfernt wird. Als Kolbenverschluß dient ein Gummistopfen mit Gärröhrchen, das mit destilliertem Wasser gefüllt ist.

Nach 7 Tagen wird der Kolbeninhalt auf Milchsäuregehalt und Restpenicillingehalt untersucht.

Die Penicillinbestimmung erfolgt durch Agardiffusionstest, kombiniert mit einem Penicillin-Test.

Es zeigt sich, daß keine Milchsäuregärung bzw. -bildung stattgefunden hat und auch der Restpenicillingehalt sich nicht wesentlich verminderte.

## Vergleichsbeispiel 2

1 kg Penicillium-Naßmyzel (stichfest) aus der Penicillin G-Produktion (TM = 10 Gew.-%, Penicillin G-Gehalt = 2520 mg/kg TM) wird ohne Fremdinfektion mit 30 g steriler Stärkezuckerlösung (Glucosegehalt = 50 Gew.-%) und 100 g Dickmilch (als Lactobazillus-Impfstoff) unter sterilen Bedingungen gemischt und das Gemisch in einem sterilen 2 l-Erlenmeyerkolben bei Raumtemperatur (23° C) unter anaeroben Bedingungen analog dem Vergleichsbeispiel 1 7 Tage stehen gelassen. Anschließend wird der Kolbeninhalt auf Milchsäuregehalt und Restpenicillingehalt untersucht.

Es zeigt sich, daß keine Milchsäuregärung bzw. -bildung stattgefunden hat und auch der Restpenicillingehalt sich nicht wesentlich verminderte. Dies bedeutet, daß die Lactobazillen der Dickmilchsäuerung in der Restpenicillin enthaltenden Naßmyzelmasse keine Milchsäuregärung bewerkstelligen können.

Die Trockenmassen (= TM) der in den Vergleichsbeispielen 1 und 2 eingesetzten Penicillium-Naßmyzele haben folgende Zusammensetzung:

91 Gew.-% organische Substanz, davon 44 Gew.-% Rohprotein,
9 Gew.-% anorganische Substanz,
Restpenicillin G-Gehalt 2350 bzw. 2520 mg/kg TM.

## Beispiel 1

0,5 kg Penicillium-Naßmyzel (stichfest) aus der Penicillin G-Produktion (TM = 18 Gew.-%, bestehend aus 91 Gew.-% org. Substanz und 9 Gew.-% anorg. Substanz, Penicillin G-Gehalt = 2060 mg/kg TM, pH-Wert = 5,6), werden mit 50 g Stärkezuckerlösung (Glucosegehalt = 50 Gew.-%) und 0,5 kg feuchter Grünmaissilage gemischt und das Gemisch in einen 2-l-Erlenmeyerkolben eingefüllt. Der Restsauerstoff in dem Kolben wird durch Stickstoffüberlagerung entfernt und der Kolben mit einem Gummistopfen mit Gärröhrchen, das mit dest. Wasser gefüllt ist, verschlossen. Nach 12 Tagen anaerobem Stehen bei 23° C wird der Kolbeninhalt auf Milchsäuregehalt, Restpenicillingehalt und pH-Wert untersucht.

Die Penicillinbestimmung erfolgt durch Agardiffusionstest, kombiniert mit einem Penicillinase-Test. Die pH-Wert-Bestimmung erfolgt mittels Glaselektrode. Der Milchsäuregehalt wird titrimetrisch ermittelt.

Analyse nach 12-tägiger Silierung:

| | |
|---|---|
| pH-Wert des Silageproduktes | 4,4 |
| Milchsäuregehalt im Silageprodukt | 3,6 Gew.-% |
| Restgehalt an Penicillin G<br>(bezogen auf die eingesetzte Myzel-Trockenmasse) | nicht nachweisbar<br>(= < 0,05 mg/kg Myzel-TM) |

Das resultierende ockerfarbige Silageprodukt ist von flüssiger Konsistenz. Es besitzt einen säuerlichen, angenehm hefig-aromatischen Geruch, enthält penicillinresistente Lactobazillen und wird als Lactobazillus-Impfmaterial für neue Rohmyzel-Silierungsansätze verwendet. Der Bakterientiter, der durch Auszählen ermittelt wird, beträgt $10^7 - 10^8$ Lactobazillus-Keime/g Feuchtsilageprodukt.

Beispiel 2

2 kg Penicillium-Naßmyzel (stichfest) aus der Penicillin G-Produktion (TM = 15 Gew.-%, bestehend aus 91 Gew.-% org. Substanz und 9 Gew.-% anorg. Substanz, Penicillin G-Gehalt = 4000 mg/kg TM, pH-Wert = 5,9) werden am Ende der Abernteleitung des Penicillin-Fermentationstanks auf einem Filtertuch in einer Schichtdicke von etwa 5 cm ausgestrichen und in dieser Form 2 Stunden lang an der atmosphärischen Luft in dem Aufarbeitungsbetrieb zum Zweck einer Lactobazillus-Infektion stehen gelassen.

1 kg dieses an der Luft bakteriell infizierten Penicillium-Naßmyzels mit den vorstehend beschriebenen Kenndaten wird mit 100 g Stärkezuckerlösung (Glucosegehalt = 50 Gew.-%) gemischt und das Gemisch in einem Erlenmeyerkolben unter anaeroben Bedingungen wie in Beispiel 1 beschrieben 18 Tage stehen gelassen, wobei eine Silierung stattfindet. Die Untersuchung des Kolbeninhalts erfolgt wie in Beispiel 1 beschrieben.

Analyse nach 18tägiger Silierung:

| | |
|---|---|
| pH-Wert des Silageproduktes | 4,3 |
| Milchsäuregehalt im Silageprodukt | 4,2 Gew.-% |
| Restgehalt an Penicillin G (bezogen auf die eingesetzte Myzel-Trockenmasse) | nicht nachweisbar (= < 0,05 mg/kg Myzel-TM) |

Das resultierende ockerfarbige Silageprodukt ist von flüssiger Konsistenz. Es besitzt einen säuerlichen, angenehm hefig-aromatischen Geruch, enthält penicillinresistente Lactobazillen, die unter dem Mikroskop in großer Anzahl sichtbar sind, und wird als Lactobazillus-Impfmaterial für neue Rohmyzel-Silierungsansätze verwendet.

Der Bakterientiter beträgt 1 bis 5 · $10^8$ Lactobazillus-Keime/g Feuchtsilageprodukt.

Beispiel 3

Silierung in einem 12 cbm fassenden zylindrischen Silierturm aus Kunststoff (GFK-Material), Ø 1 m, im Bodenteil konisch verjüngt mit Ablaßvorrichtung, oben durch lose aufliegende Abdeckplatte gegen Lufteintritt verschlossen.

10 000 kg Penicillium-Naßmyzel (stichfest) aus der Penicillin G-Produktion (TM = 15,5 Gew.-%, bestehend aus 91 Gew.-% org. Substanz und 9 Gew.-% anorg. Substanz, Penicillin G-Gehalt = 3500 mg/kg TM, pH-Wert = 5,6) + 300 kg Melasse (Zuckergehalt = 50 Gew.-%) + 400 kg penicillinresistente Lactobazillen enthaltende Impfstoff-Silage, hergestellt nach Beispiel 1, werden abwechselnd schichtweise in den Silierturm eingefüllt und der Turm mit einem lose aufliegenden Deckel verschlossen. Die Silierungsdauer beträgt 7 Tage bei 20—25°C. Die Untersuchung des Silageproduktes erfolgt wie in Beispiel 1 beschrieben.

Analyse nach 7-tägiger Silierung:

| | |
|---|---|
| pH-Wert des Silageproduktes | 4,4 |
| Milchsäuregehalt im Silageprodukt | 2,7 Gew.-% |
| Restgehalt an Penicillin G (bezogen auf die eingesetzte Myzel-Trockenmasse) | nicht nachweisbar (= < 0,05 mg/kg Myzel-TM) |

Das resultierende ockerfarbige Silageprodukt ist von flüssiger Konsistenz und ist pumpfähig. Es besitzt einen säuerlichen, angenehm hefig-aromatischen Geruch, enthält penicillinresistente Lactobazillen, die unter dem Mikroskop in großer Anzahl sichtbar sind. Der relativ niedrige Milchsäuregehalt entspricht dem ebenfalls relativ geringen Zuckeranteil, welcher der eingesetzten Rohmyzelmasse zugesetzt wurde. Das Silageprodukt ist unter üblichen anaeroben Bedingungen gut lagerungsfähig.

Beispiel 4

Silierung in einem 12 cbm fassenden Silierturm entsprechend Beispiel 3.

Um den Trockenmassegehalt des Ausgangs-Naßmyzels zu erhöhen, wird letzteres durch Nachpressen auf einer Winkler-Bandpresse weiter entwässert und mit dem resultierenden höheren TM-Gehalt wie folgt siliert:

10 000 kg nachgepreßtes Penicillium-Naßmyzel (stichfest) aus der Penicillin G-Produktion (TM = 26,5 Gew-%, bestehend aus 91 Gew.-% org. Substanz und 9 Gew.-% anorg. Substanz, Penicillin G-Gehalt = 2800 mg/kg TM, pH-Wert = 5,8) + 300 kg Melasse (Zuckergehalt = 50 Gew.-%) + 1000 kg penicillinresistente Lactobazillen enthaltende Impfstoff-Silage, hergestellt nach Beispiel 3, werden abwechselnd schichtweise in den Silierturm eingefüllt und, wie im Beispiel 3 beschrieben, 7 Tage bei 20—25° C siliert und das Silageprodukt untersucht.

Analyse nach 7tägiger Silierung:

| | |
|---|---|
| pH-Wert des Silageproduktes | 4,5 |
| Milchsäuregehalt im Silageprodukt | 3,0 Gew.-% |
| Restgehalt an Penicillin G (bezogen auf die eingesetzte Myzel-Trockenmasse) | nicht nachweisbar (= <0,05 mg/kg Myzel-TM) |

Das resultierende Silageprodukt hat praktisch gleiches Aussehen und analoge Eigenschaften wie das in dem Beispiel 3 beschriebene Silageprodukt. Es ist flüssig und pumpfähig.

Beispiel 5

Die in den Beispielen 3 und 4 beschriebene Silierung im Silierturm kann auch kontinuierlich betrieben werden. Dazu werden dem mit Silageprodukt gefüllten Silierturm täglich etwa 2 cbm fertige Silage von unten entnommen und 2 cbm frisches Rohmyzel mit Melassezusatz +10 Gew.-% penicillinresistente Lactobazillen enthaltende Impfstoff-Silage von oben zugeführt. Als Impfstoff-Silage dient die unten entnommene Silage. Die Mindestverweilzeit der zu silierenden Myzelmasse im Silierturm bei ca. 20 bis 25° C beträgt etwa 6 Tage. Das kontinuierlich gewonnene Silageprodukt hat analoge Eigenschaften wie die in den Beispielen 3 und 4 beschriebenen Silageprodukte und ist ebenfalls vollständig frei von Restgehalten an Penicillin G.

**Patentansprüche**

1. Verfahren zur Herstellung von penicillinfreien Myzelmassen aus fermentativ gebildeten, wasserhaltigen Penicillinproduktionskulturen (=Naßmyzel), die übliche Mengen an Restpenicillin enthalten, unter Eliminierung des Restpenicillins, dadurch gekennzeichnet, daß man die Restpenicillin enthaltende Penicillium-Rohmyzelmasse einer anaeroben Milchsäuregärung unter Verwendung penicillinresistenter Lactobazillen unterwirft.

2. Verfahren nach Anspruch 1, dadurch gekennzeichnet, daß man zunächst in einer Teilmenge der Penicillium-Rohmyzelmasse unter an sich bekannten Bedingungen eine anaerobe Milchsäuregärung durchführt, wobei penicillinresistente Lactobazillen erhalten werden und die Rohmyzelmasse in ein penicillinfreies Myzelsilageprodukt umgewandelt wird, welches man anschließend als Impfstoff der Hauptmenge der Rohmyzelmasse zuführt und diese einer anaeroben Milchsäuregärung gemäß Anspruch 1 unterwirft.

3. Verfahren nach Anspruch 2, dadurch gekennzeichnet, daß man der Ausgangsteilmenge der Penicillium-Rohmyzelmasse Gärfuttersilage, vorzugsweise Maissilage, als Lactobazillus-Impfstoff zuführt.

4. Verfahren nach Anspruch 2, dadurch gekennzeichnet, daß man die Ausgangsteilmenge der Penicillium-Rohmyzelmasse durch mehrstündiges offenes Stehenlassen an der Luft mit Lactobazillen impft.

5. Verfahren nach Anspruch 1, dadurch gekennzeichnet, daß man ein gemäß Anspruch 2 hergestelltes penicillinfreies Myzelsilageprodukt als penicillinresistenten Lactobazillus-Impfstoff verwendet.

6. Verfahren nach Ansprüchen 1 bis 5, dadurch gekennzeichnet, daß das resultierende penicillinfreie Myzelsilageprodukt einen pH-Wert von <4,5, vorzugsweise 4,2 bis 4,5, aufweist.

7. Verfahren nach Ansprüchen 1 bis 6, dadurch gekennzeichnet, daß die eingesetzte Penicillium-Rohmyzelmasse einen Trockenmasseanteil von 10 bis 30 Gew.-%, vorzugsweise 15 bis 30 Gew.-%, besitzt und der Rest zu 100 Gew.-% im wesentlichen aus Wasser besteht.

8. Verfahren nach Ansprüchen 1 bis 7, dadurch gekennzeichnet, daß man Penicillium-Rohmyzelmasse der Penicillin G-Produktion einsetzt.

9. Verfahren nach Ansprüchen 1 bis 8, dadurch gekennzeichnet, daß man der Penicillium-Rohmyzelmasse landwirtschaftliche Gärfuttersilagen oder deren Ausgangsbestandteile oder Getreidestroh zusetzt.

10. Verfahren nach Ansprüchen 1 bis 9, dadurch gekennzeichnet, daß der durch Lactobazillen in Milchsäure umwandelbare Zuckeranteil in der Rohmyzelmasse, bezogen auf die wasserhaltige Rohmyzelmasse, 1,5 bis 5 Gew.-% beträgt.

11. Verfahren nach Anspruch 10, dadurch gekennzeichnet, daß der Zuckeranteil aus Melassezucker besteht.

12. Verfahren nach Ansprüchen 1 bis 11, dadurch gekennzeichnet, daß man das Verfahren kontinuierlich durchführt.

13. Penicillinfreies Myzelsilageprodukt, hergestellt nach einem der Ansprüche 1 bis 12.

14. Verwendung von Myzelsilageprodukt nach Anspruch 13 als Tierfutter.

15. Verwendung von Myzelsilageprodukt nach Anspruch 13 als Rinder- und Schweinefutter.

16. Futtermittel, gekennzeichnet durch einen Gehalt an Myzelsilageprodukt nach Anspruch 13.

17. Mischfuttermittel, gekennzeichnet durch einen Gehalt an Myzelsilageprodukt nach Anspruch 13.

18. Verfahren zum Mästen von Rindern und Schweinen, dadurch gekennzeichnet, daß man sie mit Futtermitteln nach Ansprüchen 16 und 17 füttert.

19. Verwendung von Myzelsilageprodukt nach Anspruch 13 als Düngemittel.

20. Verfahren zum Düngen von Pflanzenkulturen, Kulturböden oder Vegetation tragenden Substraten, dadurch gekennzeichnet, daß man diese mit düngewirksamen Mengen von Myzelsilageprodukten nach Anspruch 13 behandelt.

## Claims

1. A process for the preparation of penicillin-free mycelium masses from water-containing penicillin production cultures (=wet mycelium) which are formed by fermentation and contain the usual amounts of residual penicillin, with elimination of the residual penicillin, which comprises subjecting the Penicillium crude mycelium mass containing residual penicillin to anerobic lactic fermentation using penicillin-resistant Lactobacilli.

2. The process as claimed in claim 1, wherein anerobic lactic fermentation is first carried out in a part amount of the Penicillium crude mycelium mass under conditions which are known per se, penicillin-resistant Lactobacilli being obtained and the crude mycelium mass being converted into a penicillin-free mycelium silage produkt, which is then passed to the main portion of the crude mycelium mass as an inoculum, the mass being subjected to anerobic lactic fermentation as claimed in claim 1.

3. The process as claimed in claim 2, wherein silage, preferably maize silage, is added, as a Lactobacillus inoculum, to the starting part amount of the Penicillium crude mycelium mass.

4. The process as claimed in claim 2, wherein the starting part amount of the Penicillium crude mycelium mass is inoculated with Lactobacilli by being left to stand open in the atmosphere for several hours.

5. The process as claimed in claim 1, wherein a penicillin-free mycelium silage product prepared as claimed in claim 2 is used as the penicillin-resistant Lactobacillus inoculum.

6. The process as claimed in any of claims 1 to 5, wherein the resulting penicillin-free mycelium silage product has a pH value of $<4.5$, preferably 4.2 to 4.5.

7. The process as claimed in any of claims 1 to 6, wherein the Penicillium crude mycelium mass employed has a dry mass content of 10 to 30% by weight, preferably 15 to 30% by weight, and the remainder to make up to 100% by weight substantially consists of water.

8. The process as claimed in any of claims 1 to 7, wherein the Penicillium crude mycelium mass from the production of penicillin G is used.

9. The process as claimed in any of claims 1 to 8, wherein agricultural silages or their starting constituents or cereal straw is added to the Penicillium crude mycelium mass.

10. The process as claimed in any of claims 1 to 9, wherein the sugar content, in the crude mycelium mass, which can be converted into lactic acid by Lactobacilli is 1.5 to 5% by weight, relative to the water-containing crude mycelium mass.

11. The process as claimed in claim 10, wherein the sugar content consists of molasses sugar.

12. The process as claimed in any of claims 1 to 11, as a continuous process.

13. A penicillin-free mycelium silage produkt prepared as claimed in any of claims 1 to 12.

14. The use of a mycelium silage product as claimed in claim 13 as animal feed.

15. The use of a mycelium silage produkt, as claimed in claim 13, as feed for cattle and pigs.

16. A feed which contains a mycelium silage produkt as claimed in claim 13.

17. A mixed feed, which contains a mycelium silage product as claimed in claim 13.

18. A method of fattening cattle and pigs, wherein the animals are fed with feeds as claimed in claim 16 or 17.

19. The use of a mycelium silage product, as claimed in claim 13, as fertilizer.

20. A prozess for fertilizing plant crops, growing soils or substrates containing vegetation, wherein these are treated with fertilizing amounts of a mycelium silage product as claimed in claim 13.

## Revendications

1. Procédé de préparation de masses mycéliennes extemptes de pénicilline à partir de cultures de production de pénicilline contenant de l'eau (=mycélium humide) qui contiennent les quantités habituelles de pénicilline résiduelle, avec élimination de la pénicilline résiduelle, caractérisé en ce qu'on soumet la masse mycélienne brute de pénicillium contenant la pénicilline résiduelle à une fermentation lactique anaérobie avec utilisation de lactobacilles résistant à la pénicilline.

2. Procédé suivant la revendication 1, caractérisé en ce qu'on effectue d'abord dans une quantité fractionnaire de la masse mycélienne brute de pénicillium, dans des conditions connues en soi, une fermentation lactique anaérobie, de sorte que l'on obtient des lactobacilles résistant à la pénicilline, et on transforme la masse de mycélium brut en un produit d'ensilage mycélien exempt de pénicilline, qui est ensuite envoyé comme substance d'inoculation à la partie principale de la masse de mycélium brut, et on soumet celle-ci à une fermentation lactique anaérobie conformément à la revendication 1.

3. Procédé suivant la revendication 2, caractérisé en ce qu'on envoie à la quantité fractionnaire initiale de la masse mycélienne brute de pénicillium un produit ensilé, de préférence un maïs ensilé, en tant que substance d'inoculation de lactobacilles.

4. Procédé suivant la revendication 2, caractérisé en ce qu'on inocule avec des lactobacilles la quantité fractionnaire initiale de la masse mycélienne brute de pénicillium par un séjour de plusieurs heures à l'air libre.

5. Procédé suivant la revendication 1, caractérisé en ce qu'on utilise un produit mycélien ensilé exempt de pénicilline préparé conformément à la revendication 2 comme substance d'inoculation de lactobacilles résistant à la pénicilline.

6. Procédé suivant les revendications 1 à 5, caractérisé en ce que le produit mycélien ensilé exempt de pénicilline obtenu présente un pH inférieur à 4,5, de préférence de 4,2 à 4,5.

7. Procédé suivant les revendications 1 à 6, caractérisé en ce que la masse mycélienne brute de pénicillium utilisée possède une proportion de masse sèche de 10 à 30% en poids, de préférence de 15 à 30% en poids, et en ce que le complément à 100% en poids se compose essentiellement d'eau.

8. Procédé suivant les revendications 1 à 7, caractérisé en ce qu'on utilise une masse mycélienne brute de pénicillium de la production de pénicilline G.

9. Procédé suivant les revendications 1 à 8, caractérisé en ce qu'on ajoute à la masse mycélienne brute de pénicillium des produits ensilés agricoles ou leurs constituants de départ, ou la paille de blé.

10. Procédé suivant les revendications 1 à 9, caractérisé en ce que la proportion de sucre transformable en acide lactique par les lactobacilles dans la masse mycélienne brute, rapportée à la masse mycélienne brute contenant de l'eau, est de 1,5 à 5% en poids.

11. Procédé suivant la revendication 10, caractérisé en ce que la fraction sucre se compose de sucre de mélasse.

12. Procédé suivant les revendications 1 à 11, caractérisé en ce qu'on effectue le procédé en continu.

13. Produit ensilé mycélien exempt de pénicilline, préparé suivant l'une des revendications 1 à 12.

14. Utilisation de produits d'ensilage mycéliens suivant la revendication 13 comme fourrage.

15. Utilisation de produits mycéliens ensilés suivant la revendication 13 comme fourrage pour bovins et porcs.

16. Fourrage, caractérisé en ce qu'il contient un produit mycélien ensilé suivant la revendication 13.

17. Fourrage mixte, caractérisé en ce qu'il contient un produit mycélien ensilé suivant la revendication 13.

18. Procédé d'engraissement de bovins et de porcs, caractérisé en ce qu'on les nourrit avec des fourrages selon les revendications 16 et 17.

19. Utilisation du produit mycélien ensilé suivant la revendication 13 comme engrais.

20. Procédé de fumure de cultures végétales, de sols de culture ou de substrats portant de la végétation, caractérisé en ce qu'on les traite avec des quantités actives comme engrais de produits mycéliens ensilés suivant la revendication 13.